(19) Europäisches Patentamt
European Patent Office
Office européen des brevets

(11) EP 1 236 708 A1

# (12) EUROPEAN PATENT APPLICATION

(43) Date of publication:
**04.09.2002 Bulletin 2002/36**

(51) Int Cl.[7]: **C07C 51/09**, C07C 51/487

(21) Application number: **02004412.9**

(22) Date of filing: **26.02.2002**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **28.02.2001 JP 2001053963**

(71) Applicant: **Sumitomo Chemical Company, Limited**
**Chuo-ku Osaka 541-8550 (JP)**

(72) Inventors:
- **Suzukamo, Gohfu**
  **Suita-shi, Osaka (JP)**
- **Sasaki, Kazuaki**
  **Ibaraki-shi, Osaka (JP)**

(74) Representative: **Henkel, Feiler, Hänzel**
**Möhlstrasse 37**
**81675 München (DE)**

## (54) Method for producing optically active chrysanthemic acid

(57) Disclosed is a method for producing an optically active chrysanthemic acid, which method is characterized by optical resolution of a chrysanthemic acid having a trans isomer ratio of not less than 70% and an optical purity of 2%e. e. to less than 10% e.e. with an optically active organic.

EP 1 236 708 A1

## Description

BACKGROUND OF THE INVENTION

The present invention relates to a method for producing an optically active chrysanthemic acid.

(+)-2,2-Dimethyl-3-(2-methyl-1-propenyl)cyclopropanecarboxylic acid known as chrysanthemum mono-carboxylic acid constitutes an acid component of synthetic pyrethroid insecticide.

The insecticidal activity of a trans-pyrethroid ester is usually higher than that of a cis-isomer. Particularly, pyrethroid esters of (+)-trans-chrysanthemum mono-carboxylic acid or chrysanthemic acid enriched with the (+)-trans-chrysanthemic acid have exhibited excellent insecticidal activity. Accordingly, an industrially advantageous method to produce (+)-trans-chrysanthemic acid or chrysanthemic acid enriched with the (+)-trans-chrysanthemic acid has been desired.

As a method of producing an optically active chrysanthemic acid derivative by using a synthetic technique, there has been known a method of reacting a (±)-trans-chrysanthemum mono-carboxylic acid or trans rich (±)-chrysanthemum mono-carboxylic acid with an optically active organic amine, which are an optically active amine, to obtain an optically active chrysanthemic acid (JP46-20382B/1971, JP54-37130B/1979, JP49-109344A/1974 and JP51-23497B/1976). However, these optical resolution methods were not always satisfactory, because of low yield of the desired optically active chrysanthemic acid.

SUMMARY OF THE INVENTION

According to the present invention, an optically active chrysanthemic acid can be obtained efficiently.

The present invention provides:

a method for producing an optically active chrysanthemic acid, which comprises optically resolving chrysanthemic acid having a trans isomer ratio of not less than 70% and an optical purity of 2% to less than 10% e.e. with an optically active organic amine (hereinafter referred to as the "present method").

Detail Description of the Invention

In the present invention, optically active chrysanthemic acid typically means (+)-trans-chrysanthemic acid, and the "optical purity" or e.e.% with respect to (+)-trans-chrysanthemic acid in the trans isomer is calculated based on the analysis using optically active column and is defined by the following equation:

100 X {[(+)-trans-chrysanthemic acid - (-)-trans-chrysanthemic acid] /

[(+)-trans-chrysanthemic acid + (-)-trans-chrysanthemic acid]}.

The optically active organic amine for optically resolving the chrysanthemic acid as defined above includes, for example, an optically active organic amine of the formula (A-1):

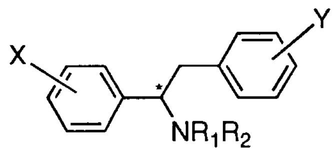

(A-1)

wherein $R_1$ and $R_2$ respectively represent

a hydrogen atom,
an alkyl group(e.g. (C1-C4)alkyl such as methyl, ethyl, n-propyl, i-propyl, n-butyl, i-butyl, t-butyl, or s-butyl),
an aralkyl group which may be substituted with a group selected from
a hydroxy,
alkoxy (e.g. (C1-C3)alkoxy such as methoxy, ethoxy, n-propoxy or
i-propoxy), and alkyl group (e.g. (C1-C3)alkyl such as methyl, ethyl, n-propyl or i-propyl);
X and Y respectively represent a hydrogen atom, a halogen atom, an alkyl group(e.g. (C1-C3)alkyl such as methyl,

ethyl, n-propyl, or i-propyl), or an alkoxyl group (e.g. (C1-C3)alkoxy such as methoxy, ethoxy, n-propoxy or i-propoxy); and

* represents an asymmetric carbon atom; or

an optically active organic amine of the formula (A-2):

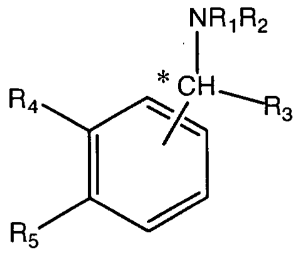

(A-2),

wherein $R_1$ and $R_2$ respectively represent

a hydrogen atom,

an alkyl group (e.g. (C1-C4) alkyl group such as methyl, ethyl, n-propyl, i-propyl, n-butyl, i-butyl, t-butyl, or s-butyl),

an aralkyl group which may be substituted with a group selected from hydroxy, alkoxy (e.g. (C1-C3)alkoxy such as methoxy, ethoxy, n-propoxy or i-propoxy), and alkyl (e.g. (C1-C3)alkyl such as methyl, ethyl, n-propyl or i-propyl);

$R_3$ represents alkyl group (e.g., (C1-C6)alkyl group such as methyl, ethyl, n-propyl, i-propyl, n-butyl, s-butyl, t-butyl, n-pentyl, n-hexyl or the like);

$R_4$ and $R_5$ represent a hydrogen atom, or $R_4$ and $R_5$ with the carbon atoms to which they are bonded form a benzene ring; and

* represents an asymmetric carbon atom.

Examples of the (C7-C10)aralkyl group represented by $R_1$ or $R_2$ include, for example, benzyl, 1-, or 2-phenethyl, phenylpropyl, phenylbutyl or the like.

Examples of the optically active organic amine includes, for example,

1-phenyl-2-(p-tolyl)ethylamine, α-(1-naphthyl)-ethylamine,
α-(2-naphthyl)-ethylamine, 1-phenylethylamine,
N-(p-hydroxybenzyl)-1-phenylethylamine,
N-(p-hydroxybenzyl)-1-(p-tolyl)ethylamine,
N-(p-hydroxybenzyl)-1-(p-isopropylphenyl)ethylamine,
N-(p-hydroxybenzyl)-1-(p-nitrophenyl)ethylamine,
N-(p-hydroxybenzyl)-1-(p-bromophenyl)ethylamine,
N-(p-hydroxybenzyl)-1-(1 -naphthyl)ethylamine,
N-(p-hydroxybenzyl)-1-(p-methoxyphenyl)ethylamine,
N-(p-hydroxybenzyl)-1-phenylpropylamine,
N-(p-hydroxybenzyl)-2-methyl-1-phenylpropylamine,
N-(2-hydroxy-3-methoxybenzyl)-1-phenylethylamine,
N-(2-hydroxy-3-methoxybenzyl)-1-(p-tolyl)ethylamine,
N-(2-hydroxy-3-methoxybenzyl)-1-(p-isopropylphenyl)ethylamine,
N-(2-hydroxy-3-methoxybenzyl)-1-(p-nitrophenyl)ethylamine,
N-(2-hydroxy-3-methoxybenzyl)-1-(p-bromophenyl)ethylamine,
N-(2-hydroxy-3-methoxybenzyl)-1-(1-naphthyl)ethylamine,
N-(2-hydroxy-3-methoxybenzyl)-1-(p-methoxyphenyl)ethylamine,
N-(2-hydroxy-3-methoxybenzyl)-1-phenylpropylamine,
N-(2-hydroxy-3-methoxybenzyl)-2-methyl-1-phenylpropylamine, and the like.

Optical resolution is performed by reacting the chrysanthemic acid with an optically active organic amine (optically active organic amine) of formula (A-1) or (A-2).

The optical resolution is usually performed in a solvent. Examples of the solvent that may be used include, aromatic hydrocarbons such as benzene, toluene, xylene or the like; aliphatic hydrocarbons such as hexane, heptane or the like; alcohols such as water, methanol, ethanol or the like; ketones such as acetone, methyl isobutyl ketone or the like; and ethers such as dioxane, tetrahydrofuran or the like; or a mixture thereof.

The amount of the solvent varies depending on the solvent and conditions of the post treatments and is not specifically limited, and an optimum amount is optionally set according to the respective conditions.
The amount of the optical active organic amine is usually from about 0.3 to 1.2 moles, and preferably from about 0.4 to 1.1 moles, per mol of the chrysanthemic acid. The optically active organic amine and chrysanthemic acid are usually dissolved in the solvent above under stirring or standing. The mixture may be heated to dissolve, if necessary.
A diastereomer salt comprising an optically active organic amine and optical isomer of chrysanthemic acid, (+)-trans-chrysanthemic acid, is usually formed to deposit from the solution on standing at an ambient temperature or by cooling the solution.
The deposited crystal may be collected as it is, or alternatively, it may be totally or partially dissolved by heating and then cooled to deposit the crystal, if necessary.
The dissolution and deposition is usually conducted at a temperature ranging from -20 to 150°C, and preferably from -10 to 100°C.
The deposited diastereomer crystal is usually separated by filtration.
The diastereomer salt separated as crystals is then decomposed with an acid or alkali and extracted to obtain the desired optically active chrysanthemic acid with higher trans ratio and optical purity. The used optically active organic amine can be recovered, if desired.
For example, decomposing of the diastereomer salt is conducted by using an aqueous hydrochloric or sulfuric acid, and extracting the resultant with an organic solvent. The optically active organic amine used for optical resolution can be recovered by making the aqueous layer weak alkali and extracting it.
Alternatively, the optical active organic amine can be recovered by decomposing the disatereomer salt thus obtained with an aqueous base such as sodium hydroxide, and extracting the resultant with an organic solvent in a weak alkali condition. Thereafter, the aqueous layer is acidified and extracted, thereby the desired optically active chrysanthemic acid is recovered.
The optical active organic amine recovered by these methods can be reused.
The chrysanthemic acid to be resolved by the present method can be produced by reacting 2,5-dimethyl-2,4-hexadiene with diazoacetate of the formula (I):

$$N_2CHCO_2R_6 \qquad (I)$$

wherein $R_6$ represents a alkyl group or a cycloalkyl group, in the presence of an asymmetric metal complex to produce an optically active chrysanthemic acid ester; and hydrolyzing the resulting chrysanthemic acid ester with an alkali or acid.
The alkyl group of diazoacetate of formula (I) used in the above reaction includes, for example, (C1-C6)alkyl group such as methyl, ethyl, n-propyl, iso-propyl, n-butyl, iso-butyl, t-butyl, pentyl, or hexyl, and the cycloalkyl includes a (C5-C6)cycloalkyl group such as cyclopentyl or cyclohexyl group.
The asymmetric metal complex includes asymmetric copper complex, asymmetric cobalt complex, asymmetric ruthenium complex, asymmetric rhodium complex, asymmetric palladium complex, or the like. The asymmetric metal complex usually comprises the metal and an optically active ligand (hereinafter referred to as "an optically active ligand") (Pure & Appl. Chem., Vol. 57, No. 12, 1839, 1985, Tetrahedron Lett., 32, 7373 (1991), Tetrahedron Lett., 35, 7985 (1994)).
Preferred is the asymmetric copper complex, which can be usually prepared from a copper compound and an optically active ligand.
The copper compound includes, for example, mono- or divalent copper compounds such as copper naphthenate, copper trifluoromethanesulfonate, copper acetate, copper bromide, copper chloride, and copper sulfate. These compounds may be used alone or in combination thereof.
The asymmetric cobalt complex, asymmetric ruthenium complex, asymmetric rhodium complex, and asymmetric palladium complex can be prepared from a cobalt compound, ruthenium compound, rhodium compound, and palladium compound, and the optically active ligand respectively.
Examples of the cobalt compound, ruthenium compound, rhodium compound, and palladium compound include, for example, acetate, trifluoromethanesulfonate and halide (e.g. chloride, bromide, iodide) of the metals.
The optically active ligand includes, for example, an optically active bisoxazoline compound, an optically active salicylideneamino alcohol compound, an optically active diamine compound, an optically active semicorrin compound and an optically active camphor compound.
Preferred examples thereof include the optically active salicylideneamino alcohol compound, the optically active bisoxazoline compound and the optically active ethylenediamine compound.

The optically active bisoxazoline compound typically include an optically active bisoxazoline compound of the formula (L-1):

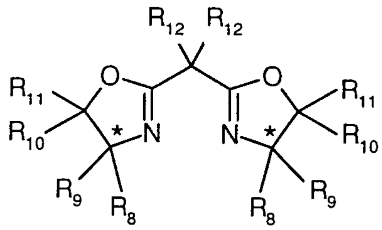

(L-1)

wherein $R_8$ and $R_9$ are different and independently represent

a hydrogen atom, an alkyl group(e.g. (C1-C6)alkyl such as methyl, ethyl, n-propyl, iso-propyl, n-butyl, iso-butyl, t-butyl, pentyl, or hexyl), a cycloalkyl group (e.g. (C3-C7)cycloalkyl such as cyclopropyl, cyclobutyl, cyclopentyl, cyclopentyl, cyclohexyl, or cycloheptyl), a phenyl or benzyl group which may be substituted;
$R_{10}$ and $R_{11}$ represent
a hydrogen atom, an alkyl group(e.g. (C1-C6)alkyl such as methyl, ethyl, n-propyl, iso-propyl, n-butyl, iso-butyl, t-butyl, pentyl, or hexyl),
a cycloalkyl group(e.g. (C3-C7)cycloalkyl such as cyclopropyl, cyclobutyl, cyclopentyl, cyclopentyl, cyclohexyl, or cycloheptyl)
a phenyl or aralkyl group which may be substituted; or
$R_{10}$ and $R_{11}$ are bonded at their terminals to form an alkylene group(e.g. (C2-C6)alkylene group such as methylene, dimethylene, trimethylene, tetramethylene, pentamethylene, or hexamethylene);
$R_{12}$ represents a hydrogen atom or an (C1-C6)alkyl group(e.g. methyl, ethyl, n-propyl, iso-propyl, n-butyl, iso-butyl, t-butyl, pentyl, hexyl); and
* represents an asymmetric carbon atom.

The aralkyl group include a (C3-C7)aralkyl group as defined above for the $R_1$ or $R_2$ of the optically active organic amine compound.

The optically active bisoxazoline compound (L-1) includes, for example, 2,2' -methylenebis[(4R)-phenyl-2-oxazoline],

2,2' -methylenebis[(4R)-4-isopropyl-2-oxazoline],,
2,2' -methylenebis[(4R)-4-t-butyl-2-oxazoline],,
2,2' -methylenebis[(4R)-4-i-butyl-2-oxazoline],,
2,2' -methylenebis[(4R)-4-benzyl-2-oxazoline],,
2,2' -methylenebis[(4R)-4-t-butyl-2-oxazoline],,
2,2' -methylenebis[(4R, 5S)-4,5-diphenyl-2-oxazoline],
2,2' -methylenebis[(4R)-4-phenyl-5,5-dimethyl-2-oxazoline],
2,2' -methylenebis[(4R)-4-phenyl-5,5-diethyl-2-oxazoline],
2,2' -methylenebis[(4R)-4-phenyl-5,5-di-n-propyl-2-oxazoline],
2,2' -methylenebis[(4R)-4-phenyl-5,5-di-i-propyl-2-oxazoline],
2,2' -methylenebis[(4R)-4-phenyl-5,5-dicyclohexyl -2-oxazoline],
2,2' -methylenebis[(4R)-4-phenyl-5,5-diphenyl-2-oxazoline],
2,2' -methylenebis[(4R)-4-phenyl-5,5-di-(2-methylphenyl)-2-oxazoline],
2,2' -methylenebis[(4R)-4-phenyl-5,5-di-(3-methylphenyl)-2-oxazoline],
2,2' -methylenebis[(4R)-4-phenyl-5,5-di-(4-methylphenyl)-2-oxazoline],
2,2' -methylenebis[(4R)-4-phenyl-5,5-di-(2-methoxyphenyl)-2-oxazoline],
2,2' -methylenebis[(4R)-4-phenyl-5,5-di-(3-methoxyphenyl)-2-oxazoline],
2,2' -methylenebis[(4R)-4-phenyl-5,5-di-(4-methoxyphenyl)-2-oxazoline],
2,2' -methylenebis[(4R)-4-phenyl-2-oxazoline-5-spiro-1'-cyclobutane],
2,2' -methylenebis[(4R)-4-phenyl-2-oxazoline-5-spiro-1'-cyclopentane],
2,2' -methylenebis[(4R)-4-phenyl-2-oxazoline-5-spiro-1'-cyclohexane],
2,2' -methylenebis[(4R)-4-phenyl-2-oxazoline-5-spiro-1'-cycloheptane],
2,2' -isopropylidenebis[(4R)-4-phenyl-2-oxazoline],

2,2' -isopropylidenebis[(4R)-4-isopropyl-2-oxazoline],
2,2' -isopropylidenebis[(4R)-4-t-butyl-2-oxazoline],
2,2' -isopropylidenebis[(4R)-4-i-butyl-2-oxazoline],
2,2' -isopropylidenebis[(4R)-4-benzyl-2-oxazoline],
2,2'-isopropylidenebis[(4R,5S)-4,5-diphenyl-2-oxazoline], and the like. and compounds wherein (4R) in the above respective compounds is replaced by (4S).

The optically active salicylideneamino alcohol compound includes a compound of the formula (L-2):

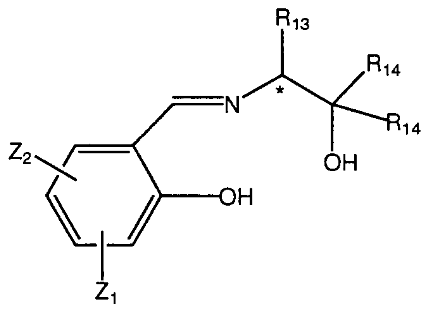

(L-2)

wherein $R_{13}$ represents

an alkyl group(e.g. (C1-C10)alkyl such as methyl ethyl, propyl, butyl, pentyl, hexyl, octyl, nonyl, or decyl),
a cycloalkyl group(e.g. (C5-C7)cycloalkyl such as cyclopentyl, cyclohexyl or cycloheptyl),
an aryl (e.g. (C6-C10)aryl such as phenyl or naphtyl ) or aralkyl (e.g. (C7-C10)aralkyl group) group which may be substituted with a group selected from
an alkyl group (e.g. (C1-C8)alkyl such as methyl, ethyl, propyl, butyl, pentyl, hexyl, heptyl, or octyl),
an alkoxy group (e.g. (C1-C8)alkoxy such as methoxy, ethoxy, propoxy, butoxy, pentyloxy, hexyloxy, heptyloxy, or octyloxy),
a cycloalkoxy group (e.g. (C3-C7)cycloalkoxy such as cyclopropyloxy, cyclobutyloxy, cyclopentyloxy, cyclohexyloxy, or cycloheptyloxy),
an aryloxy group(e.g. (C6-C10)aryloxy such as phenoxy, naphthyloxy or the like), and
an aralkyloxy group (e.g. a (C7-C10) aralkyl such as benzyl, 1-, or 2-phenethyl, pehnylpropyl, phenylbutyl or the like);

$R_{14}$ represents

a hydrogen atom,
an alkyl group (e.g. (C1-C6)alkyl such as methyl, ethyl, propyl, butyl, pentyl, or hexyl),
a cycloalkyl group (e.g. (C4-C6)cycloalkyl such as cyclobutyl, cyclopentyl, or cyclohexyl),
a phenyl group which may be substituted a group selected from
an alkyl group (e.g. (C1-C8)alkyl such as methyl, ethyl, propyl, butyl, pentyl, hexyl, heptyl, or octyl),
an alkoxy group(e.g. (C1-C8)alkoxy such as methoxy, ethoxy, propoxy, butoxy, pentyloxy, hexyloxy, heptyloxy, or octyloxy),
a cycloalkoxy group(e.g. (C4-C8)cycloalkoxy such as cyclobutyloxy, cyclopentyloxy, cyclohexyloxy, cycloheptyloxy, or cyclooctyloxy),
an aryloxy group(e.g. (C6-C10)aryloxy such as phenyl or naphthyl), and an aralkyloxy group;
a benzyl group; or
two $R_{14}$ groups form an alkylene group(e.g. (C3-C6)alkylene group such as trimethylene, tetramethylene, pentamethylene or hexamethylene);
$Z_1$, and $Z_2$ independently represent a hydrogen atom, a halogen atom,
an alkyl group(e.g. (C1-C3)alkyl such as methyl, ethyl, or propyl ),
an alkoxy group(e.g. (C1-C3)alkoxy such as methoxy, ethoxy, or propoxy), an aralkyl group,
an aryl group(e.g. (C6-C10)aryl group such as phenyl or naphthyl),
an acyl group(e.g. (C2-C4)alkanoyl such as acetyl, propanoyl, or butanoyl, or benzoyl),
an ester group(e.g. (C1-C2)alkoxy-carbonyl such as methoxycarbonyl, or ethoxycarbonyl) or a nitro group; and

* represents an asymmetric carbon atom.

The aralkyl group include a (C3-C7)aralkyl group as defined above for the $R_1$ or $R_2$ of the optically active organic amine compound. The aralkyloxy group include a (C3-C7)aralkyloxy group having the same (C3-C7)aralkyl group as defined above.

$R_{13}$ of the preferred optically active salicylideneamino alcohol compound (L-2) includes, for example, methyl, ethyl, iso-propyl, iso-butyl, t-butyl, benzyl and phenyl groups.

Specific examples of the preferred group represented by $R_{14}$ include, for example, methyl, ethyl, iso-propyl, iso-butyl, t-butyl and benzyl groups, and phenyl group which may be substituted with an alkyl group, or an alkoxy group.

Specific examples of the compound of the formula (L-2) includes (R) isomer or (S) isomer of the following compounds:

N-salicylidene-2-amino-1,1-diphenyl-1 -propanol,
N-salicylidene-2-amino-1,1-di(2-methoxyphenyl)-1-propanol,
N-salicylidene-2-amino-1,1-di(2-isopropoxyphenyl)-1 -propanol,
N-salicylidene-2-amino-1,1-di(2-n-butoxy-5-t-butylphenyl)-1 -propanol,
N-salicylidene-2-amino-1,1-diphenyl-3-phethyl-1-propanol,
N-salicylidene-2-amino-1,1-di(2-methoxyphenyl)-3-phethyl-1-propanol,
N-salicylidene-2-amino-1,1-di(2-isopropoxyphenyl)-3-phethyl-1-propanol,
N-salicylidene-2-amino-1,1-di(2-n-butoxy-5-t-butylphenyl)-3-phethyl-1 -propanol,
N-salicylidene-2-amino-1,1-dip(2-methoxthenyl)-3-methyl-1-butanol,
N-(3-fluorosalicylidene)-2-amino-1,1-di(2-n-butoxy-5-t-butylphenyl)- 1-propanol,
N-(3-fluorosalicylidene)-2-amino-1,1-di(2-octyloxy-5-t-butylphenyl)-1-propanol,
N-(3-fluorosalicylidene)-2-amino-1,1-di(2-n-butoxy-5-t-butylphenyl)-3-phenyl-1 - propanol,
N-(3-fluorosalicylidene)-2-amino-1,1 -di(2-methoxyphenyl)-1 -propanol,
N-(3-fluorosalicylidene)-2-amino-1,1-dilphenyl-1-propanol,
N-(3-fluorosalicylidene)-2-amino-1,1-di(2-benzyloxy-5-t-butylphenyl)-3-(4-isoprop oxyphenyl)-1-propanol,
N-(3-fluorosalicylidene)-2-amino-1,1-diphenyl-3-phenyl-1-propanol,
N-(3-fluorosalicylidene)-2-amino-1,1-(2-methoxyphenyl)-3-menyl-1 -butanol,
N-(5-nitrosalicylidene)-2-amino-1,1-diphenyl-1 -propanol,
N-(5-nitrosalicylidene)-2-amino-1,1-diphenyl-3-phenyl-1-propanol,
N-(5-nitrosalicylidene)-2-amino-1,1-di(2-butoxy-5-t-butylphenyl)-3-phenyl-1 - propanol,
N-(5-nitrosalicylidene)-2-amino-1,1-di(2-butoxy-5-t-butylphenyl)-1-propanol,
N-(5-nitrosalicylidene)-2-amino-1,1-di(2-benzyloxy-5-methylphenyl)-3-(4-isopropoxyphenyl-1-propanol,
N-(5-nitrosalicylidene)-2-amino-1,1-di(2-methoxyphenyl)-1-propanol,
N-(5-nitrosalicylidene)-2-amino-1,1-di(2-t-butyl-4-methylphenyl)-3-phenyl-1 -propanol,
N-(5-nitrosalicylidene)-2-amino-1,1-di(4-t-butylphenyl)-1-propanol,
N-(5-nitrosalicylidene)-2-amino-1,1-di(2-mrthoxyphenyl)-3-methyl-1-butanol,
N-(5-bromosalicylidene)-2-amino-1,1-diphenyl-1-proanol,
N-(5-bromosalicylidene)-2-amino-1,1-di(2-methoxyphenyl)-1-proanol,
N-(3,5-dibromosalicylidene)-2-amino-1,1-diphenyl-1 -proanol,
N-(3,5-dichlorosalicylidene)-2-amino-1,1-diphenyl-1-proanol,
N-(3-t-butylsalicylidene)-2-amino-1,1-diphenyl-1 -proanol,
N-(3,5-di-t-butylsalicylidene)-2-amino-1,1-di(2-methoxyphenyl)-1-proanol,
N-(3,5-di-nitrosalicylidene)-2-amino-1,1-diphenyl-1-proanol,
N-(3-methoxy-5-nitrosalicylidene)-2-amino-1,1-diphenyl-1-proanol, and the like.

The optically active ethylenediamine alcohol compound includes a compound of the formula (L-3):

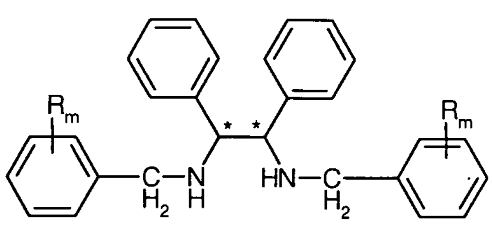

(L-3)

wherein R represents a hydrogen atom or an alkyl group(e.g. (C1-C4)alkyl such as methyl, ethyl, propyl, or butyl),

m represents an integer of 1 to 5, and
* represents an asymmetric carbon atom.

Specific compound of the optically active ethylenediamine compound (L-3) includes, for example, compounds wherein R is a hydrogen atom, a methyl group, or an ethyl group, and m is an integer of 1 to 3.
Specific examples thereof includes bis[N-(2,4,6-trimethylphenyl)methyl-(1R),(2R)-diphenylethylenediamine and an antipode thereof having (1S), (2S) configuration.
The asymmetric copper complex can be prepared by mixing the above copper compound with the optically active ligand in a solvent.
The solvent used herein includes, for example, aromatic hydrocarbons such as toluene and xylene and aliphatic halogenated hydrocarbons such as dichloromethane and dichloroethane. Furthermore, 2,5-dimethyl-2,4-hexadiene may also be used as the solvent.
The solvent is usually used in an amount of 5 to 200 parts by weight per 1 part by weight of the copper compound.
The amount of the optically active ligand is usually from about 0.8 to 5 moles, and preferably from about 1 to 2 moles, per mol of the copper compound.
In view of the reaction yield, water is preferably excluded in the above reaction.
The above reaction temperature is not specifically limited. The reaction is usually carried out at the temperature within a range from about 0 to 100°C.
In the present invention, when the complex is prepared by using a divalent copper compound, an object can be sufficiently attained even if the divalent copper compound is not reduced to form a monovalent copper compound by using a reducing agent such as phenylhydrazine.
For example, the reaction of the copper compound with the optically active ligand is usually carried out in the atmosphere of an inert gas such as argon and nitrogen.
The asymmetric copper complex can be obtained in such a manner, but the copper complex may be isolated or can be used as it is in the reaction between 2,5-dimethyl-2,4-hexadiene and the diazoacetate (I) without being isolated.
The amount of the asymmetric copper complex used in the reaction between 2,5-dimethyl-2,4-hexadiene and the diazoacetates (I) is usually from about 0.00001 to 0.02 mol, and preferably from about 0.00002 to 0.001 mol, per mol of the diazoacetate (I).
Specific method of reacting 2,5-dimethyl-2,4-hexadiene with the diazoacetate (I) in the presence of the asymmetric copper complex includes, for example, a method adding the diazoacetate (I) dissolved in a solvent to a mixture of the asymmetric copper complex obtained as described above and 2,5-dimethyl-2,4-hexadiene.
The solvent includes, for example, halogenated hydrocarbons such as dichloromethane, 1,2-dichloroethane, chloroform and carbon tetrachloride; aliphatic hydrocarbons such as hexane, heptane and cyclohexane; aromatic hydrocarbons such as benzene, toluene and xylene; and esters such as methyl acetate and ethyl acetate. It is also possible to use 2,5-dimethyl-2,4-hexadiene as the solvent. These solvents can also be used in combination.
The solvent is usually used in an amount of 1 to 50 parts by weightt, and preferably 5 to 30 parts by weight per 1 part by weight of the diazoacetate (I).
The reaction between 2,5-dimethyl-2,4-hexadiene and the diazoacetate (I) is usually carried out in an atmosphere of an inert gas such as argon and nitrogen.
2,5-dimethyl-2,4-hexadine is usually used in the amount of 1 to 50 moles, and preferably 5 to 30 moles, per mol of the diazoacetate (I).
In view of the reaction yield, water is preferably excluded in the above reaction.
The above reaction temperature is not specifically limited. The reaction can be carried out at the temperature of not more than a boiling point of the solvent when using the solvent, but is usually carried out at the temperature within a range from about -10 to 120°C, and preferably from 0 to 100°C.
The optically active chrysanthemic acid esters with considerably good purity can be obtained in the above reaction by distilling off the solvent, but can be optionally isolated by a conventional method such as distillation and column chromatography, if necessary.
The ester residue of the optically active chrysanthemic acid esters obtained by the above reaction includes, for example, methyl, ethyl, n-propyl, i-propyl, i-butyl, t-butyl, pentyl, hexyl and cyclohexyl groups.
The resulting optically active chrysanthemic acid esters can be converted into the corresponding chrysanthemic acid by contacting with an acid or an aqueous alkali solution (hydrolysis step). The amount of an alkali compound of the aqueous alkali solution used is usually from 1 to 2 moles, and preferably from 1 to 1.5 moles, per mol of the chrysanthemic acid esters.

The chrysanthemic acid, particularly having a trans isomer ratio of not less than 70% and an optical purity of 2% e.e to less than 10% e.e. thus obtained may be used in the optical resolution as it is, or it may be prepared from chrysanthemic acid enriched with the trans isomer and (+)-chrysanthemic acid. Chrysanthemic acid having trans isomer ratio of 75 to 98% and 2.5% e.e. to less than 10% e.e. is preferably used in the present method.
Such chrysanthemic acid enriched with the trans isomer can be obtained, for example, by reacting (-)-cis chrysanthemic acid or chrysanthemic acid enriched with a (-)-trans isomer with t-butyl hydroperoxide and aluminum bromide in the presence of a toluene solvent (see JP5-37137B/1993). The trans isomer ratio of the chrysanthemic acid enriched with the trans isomer used is not less than 80%, and preferably not less than 85%.
According to the present invention, there can be produced chrysanthemic acid having more improved trans/cis ratio and more improved optical purity in an industrially advantageous manner

Examples

The following Examples further illustrate the present invention in detail but are not to be construed to limit the scope thereof.

<u>Example 1</u>

To 30 g of chrysanthemic acid having an optical purity of 7.9% e.e. with respect to trans isomer and 26.8% e.e. with respect to cis isomer, and a trans/cis ratio of 80/20,120 g of toluene was added thereto and dissolved under stirring. Then, 14.8 g of (S)-1-phenyl-2-(p-tolyl)ethylamine (optically active organic amine ) was added thereto and dissolved under heating. After cooling to room temperature, the deposited crystals were collected by filtration, washed with toluene and then dissolved in an aqueous 5% sodium hydroxide. After the optically active organic amine was extracted with toluene, the aqueous layer was acidified with aqueous 5% sulfuric acid and extracted with toluene. Then, toluene was distilled off to obtain 10.3 g of chrysanthemic acid having a trans/cis ratio of 82/18 and an optical purity of 95% e.e. with respect to (+)-trans isomer and 98% e.e. with respect to (+)-cis isomer (yield: 37.6%).

<u>Example 2</u>

To 30 g of chrysanthemic acid having an optical purity of 3.7% e.e. with respect to (+)-trans isomer and 21.4 % e.e. with respect to (+)-cis isomer and a trans/cis ratio of 79/21, 180 g of toluene was added thereto and dissolved under stirring. Then, 21.2 g of (S)-1 -phenyl-2-(p-tolyl)ethylamine (optically active organic amine) was added to the solution and dissolved under heating. After cooling to room temperature, the deposited crystals were collected by filtration, washed with toluene and then dissolved in an aqueous 5% sodium hydroxide. After the optically active organic amine was extracted with toluene, the aqueous layer was acidified with aqueous 5% sulfuric acid and extracted with toluene. Then, the toluene was distilled off to obtain 10.3 g of chrysanthemic acid having a trans/cis ratio of 82/18 and an optical purity of 95% e.e with respect to (+)-trans isomer and 98 % e.e. with respect to (+)-cis isomer. (yield: 34.3%).

<u>Example 3</u>

3.62 mg of copper complex prepared from 1.6 mg (0.008 mmol) of copper acetate monohydrate and 3.44 mg of (R)-N-salicylidene-2-amino-1,1-di(2-methoxyphenyl)propanol, and 6.0 g (55 mmol) of 2,5-dimethyl-2,4-hexadiene were charged in a 50 ml Schlenk's tube wherein the atmosphere is substituted with nitrogen, followed by the addition of 0.86 mg of phenylhydrazine, and 1.14 g (10 mmol) of ethyl diazoacetate was added dropwise at 80°C over 2 hours. After the completion of the dropwise addition of ethyl diazoacetate, the mixture was further stirred at 80°C for 1 hour. The amount of chrysanthemic acid ethyl ester formed was found by gas chromatography tobe 1.76 g and the yield was 90.0% based on ethyl diazoacetate andthe trans/cis ratio was 59/41. After 2,5-dimethyl-2,4-hexadiene (boiling point: 51°C/30 mmHg) was distilled off from the reaction mixture, 1 g of the concentrated solution was taken out and mixed with 10 ml of an aqueous 1N sodium hydroxide solution and 5 ml of ethanol to effect hydrolysis in alkaline solution under stirring at 100°C for 1 hour. The resulting chrysanthemic acid was reacted with I-menthol and the formed diastereomers were analyzed by gas chromatography to find that the optical purity of the (+)-trans isomer was 35 % e.e., whereas, the optical purity of the (+)-cis isomer was 35 % e.e.
Chrysanthemic acid obtained by hydrolyzing the ethyl chrysanthemate with 1 N sodium hydroxide was mixed with racemic chrysanthemic acid enriched with trans isomer (trans/cis ratio: 95/5) in a weight ratio of 53 : 57 to give chrysanthemic acid having a trans/cis ratio of 81/19, optical purity with respect to the trans isomer: 9.6% e.e and optical purity with respect to cis isomer: 31% e.e. 109.8 g of toluene was then added to 18.3 g of the chrysanthemic acid, and the mixture was dissolved under stirring. Then, 14.4 g of (S)-1-phenyl-2-(p-tolyl)ethylamine ( optically active organic amine) was added to the solution and dissolved under heating. After cooling to room temperature, the deposited crystals

were collected by filtration, washed with toluene and then dissolved in an aqueous 5% sodium hydroxide. After the optically active organic amine was extracted with toluene, the aqueous layer was acidified with aqueous 5% sulfuric acid and extracted with toluene to obtain 7.3 g of chrysanthemic acid having a trans/cis ratio of 81/19 and an optical purity of 91% e.e. with respect to (+)-trans isomer and 95% e.e. with respect to (+)-cis isomer (yield: 40.1%).

Comparative Example 1

To 100 g of a racemic chrysanthemic acid (trans/cis ratio: 75/25), 390g of toluene was added, and the mixture was dissolved with stirring. Then, 47.0g of (S)-1-phenyl-2-(p-tolyl)ethylamine (optically active organic amine) was added and dissolved with heating. After cooling to room temperature, the deposited crystal was collected by filtration, washed with toluene and then dissolved in an aqueous 5% sodium hydroxide. After the agent for optical resolution was extracted with toluene, the aqueous layer was acidified with aqueous 5% sulfuric acid and extracted with toluene to obtain 20.8 g of chrysanthemic acid having a trans/cis ratio of 80/20 and an optical purity of the (+)-trans isomer was 96% e.e. and that of (+)-cis isomer was 98% e.e.(yield: 20.8%).

Comparative Example 2

To 16.3 g of a racemic chrysanthemic acid (trans/cis ratio: 79/21), 65.0g of toluene was added, and the mixture was dissolved with stirring. Then, 16.0 g of (S)-α-(1-naphthyl)ethylamine (optically active organic amine) and 1.3 g of water were added and dissolved with heating. After cooling to room temperature, the deposited crystal was collected by filtration, washed with toluene and then dissolved in an aqueous 5% sodium hydroxide. After the agent for optical resolution was extracted with toluene, the aqueous layer was acidified with aqueous 5% sulfuric acid and extracted with toluene to obtain 4.13 g of chrysanthemic acid having a trans/cis ratio of 98/2 and an optical purity of 97% e.e. with respect to (+)-trans isomer and 73%e.e. with respect to (+)-cis isomer (yield: 25.3%).

Example 4

To 143 g of chrysanthemic acid having an optical purity of 2.7% e.e. with respect to (+)-trans isomer and 17.6 % e.e. with respect to (+)-cis isomer and a trans/cis ratio of 78/22, 502 g of toluene was added thereto and dissolved under stirring. Then, 95.4 g of (S)-1-phenyl-2-(p-tolyl)ethylamine (optically active organic amine) was added to the solution and dissolved under heating. After cooling to room temperature, the deposited crystals were collected by filtration, washed with toluene and then dissolved in an aqueous 5% sodium hydroxide. After the optically active organic amine was extracted with toluene, the aqueous layer was acidified with aqueous 5% sulfuric acid and extracted with toluene. Then, the toluene was distilled off to obtain 40.8 g of an optically active chrysanthemic acid having a trans/cis ratio of 81/19 and an optical purity of 95% e.e with respect to (+)-trans isomer and 97 % e.e. with respect to (+)-cis isomer. (yield: 33.6%).

## Claims

1. A method for producing an optically active chrysanthemic acid, comprising optically resolving a chrysanthemic acid having a trans isomer ratio of not less than 70% and an optical purity of 2% e.e. to less than 10% e.e. with an optically active organic amine.

2. A method according to claim 1, wherein the optical resolution is conducted by reacting the chrysanthemic acid with the optically active organic amine to produce a diastereomer salt comprising (+)-trans-chrysanthemic acid and the optically active organic amine, and decomposing the salt with an acid or base.

3. A method according to claim 1 or 2, wherein the chrysanthemic acid to be resolved is a chrysanthemic acid having a trans isomer ratio of 75% to 98% and an optical purity of 2.5% e.e. to less than 10% e.e.

4. A method according to claim 1 or 2, wherein said optically active amine is an optically active amine of formula (A-1):

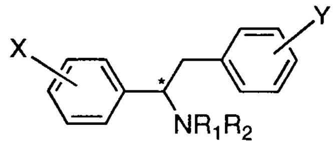

(A-1),

wherein $R_1$ and $R_2$ respectively represent

a hydrogen atom,
an alkyl group,
an aralkyl group which may be substituted with a group selected from
a hydroxy, alkoxy, and alkyl group;
X and Y respectively represent a hydrogen atom, a halogen atom, an alkyl group, or an alkoxyl group; and
* represents an asymmetric carbon atom; or
an optically active amine of formula (A-2):

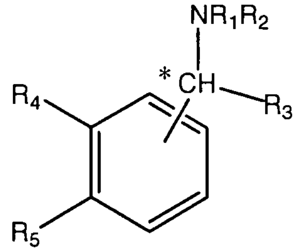

(A-2),

wherein $R_1$ and $R_2$ respectively represent

a hydrogen atom,
an alkyl group,
an aralkyl group which may be substituted with a group selected from hydroxy, alkoxy, and alkyl; or
$R_3$ represents alkyl group;
$R_4$ and $R_5$ represent a hydrogen atom, or $R_4$ and $R_5$ with the carbon atoms to which they are bonded form a benzene ring; and
* represents an asymmetric carbon atom.

**5.** A method for producing an optically active chrysanthemic acid, comprising the steps of
reacting 2,5-dimethyl-2,4-hexadiene with diazoacetates of the formula (I):

$$N_2CHCO_2R_6 \quad (I)$$

wherein $R_6$ represents an alkyl group or a cycloalkyl group, in the presence of an asymmetric metal complex to produce an optically active chrysanthemic acid esters,

reacting the chrysanthemic acid esters with an acid or base to form an chrysanthemic acid having a trans isomer ratio of not less than 70% and an optical purity of 2% e.e. to less than 10% e.e., and

optically resolving the chrysanthemic acid with an optically active organic amine of formula (A-1)

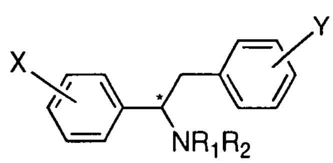

(A-1),

wherein $R_1$ and $R_2$ respectively represent

a hydrogen atom,
an alkyl group,
an aralkyl group which may be substituted with a group selected from
a hydroxy, alkoxy, and alkyl group;,
X and Y respectively represent a hydrogen atom, a halogen atom, an alkyl group or an alkoxyl group; and
* represents an asymmetric carbon atom; or
an optically active amine of formula (A-2):

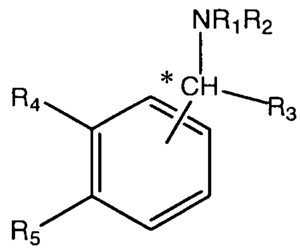

(A-2),

wherein $R_1$ and $R_2$ respectively represent

a hydrogen atom,
an alkyl group,
an aralkyl group which may be substituted with a hydroxy, alkoxy, or alkyl group;
$R_3$ represents an alkyl group,
$R_4$ and $R_5$ represents a hydrogen atom, or $R_4$ and $R_5$ with the carbon atoms to which they are bonded form a benzene ring, and
* represents an asymmetric carbon atom.

6. A method according to claim 5, wherein the metal of the asymmetric metal complex is copper, cobalt, rhodium, ruthenium, or palladium.

7. A method according to claim 5, wherein the asymmetric metal complex is an asymmetric copper complex.

8. A method according to claim 5, wherein the asymmetric metal complex is an asymmetric metal complex comprising a metal and a ligand

9. A method according to claim 8, wherein the ligand is an optically active bisoxazoline compound of the formula (L-1):

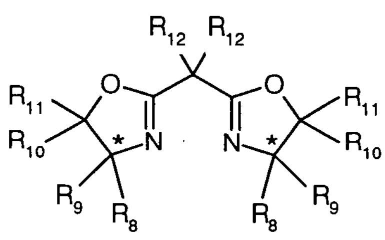

(L-1)

wherein $R_8$ and $R_9$ are different and independently represent

a hydrogen atom, an alkyl group, a cycloalkyl group, a phenyl or benzyl group which may be substituted,
$R_{10}$ and $R_{11}$ represent a hydrogen atom, an alkyl group, a cycloalkyl group, a phenyl or aralkyl group which may be substituted, or
$R_{10}$ and $R_{11}$ are bonded at their terminals to form an alkylene group;
$R_{12}$ represents a hydrogen atom or an alkyl group; and
* represents an asymmetric carbon atom.

**10.** A method according to claim 8, wherein the ligand is an optically active salicylideneamino alcohol compound of formula (L-2):

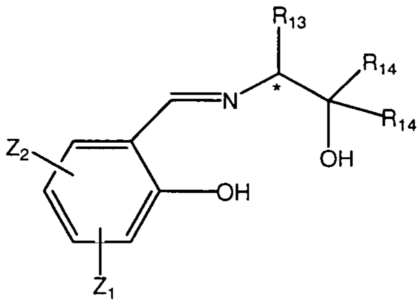

(L-2),

wherein $R_{13}$ represents

an alkyl group,
a cycloalkyl group, or
an aryl or aralkyl group which may be substituted with a group selected from an alkyl group, an alkoxy group, a cycloalkoxy group, an aryloxy group, and an aralkyloxy group;
$R_{14}$ represents
a hydrogen atom,
an alkyl group,
a cycloalkyl group, or
a phenyl group which may be substituted a group selected from an alkyl group, an alkoxy group, a cycloalkoxy group, an aryloxy group, and an aralkyloxy group,
a benzyl group; or
two $R_{14}$ groups form an alkylene group;
$Z_1$, and $Z_2$ independently represent a hydrogen atom, a halogen atom, an alkyl group, an alkoxy group, an aralkyl group, an aryl group, an acyl group, an ester group, or a nitro group, and
* represents an asymmetric carbon atom.

**11.** A method according to claim 8, wherein the ligand is an optically active ethylenediamine compound of formula (L-3):

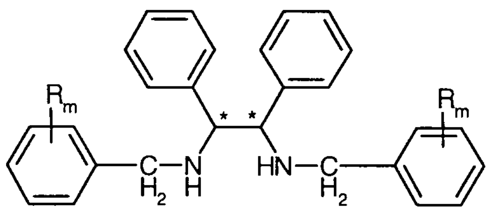

(L-3),

wherein R represents a hydrogen atom or an alkyl group; m represents an integer of 1 to 5; and * represents an asymmetric carbon atom.

European Patent Office

# EUROPEAN SEARCH REPORT

Application Number
EP 02 00 4412

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | EP 0 933 349 A (SUMITOMO CHEMICAL CO) 4 August 1999 (1999-08-04) * the whole document * | 1-11 | C07C51/09 C07C51/487 |
| D,X | KANEMASA S ET AL: "C2-Symmetric 1,2-Diamine/Coppe(II) Trifluoromethanesulfonate Complexes as Chiral Catalysts. Asymmetric Cyclopropanations of Styrene with Diazo Esters" TETRAHEDRON LETTERS, ELSEVIER SCIENCE PUBLISHERS, AMSTERDAM, NL, vol. 35, no. 43, 1994, pages 7985-7988, XP002100966 ISSN: 0040-4039 * abstract * * scheme 2 * | 5-11 | |
| X | LOWENTHAL R E ET AL: "Asymmetric Copper-Catalyzed Cyclopropanation ofTtrisubstituted and Unsymmetrical cis-1,2-Disubstituted Olefins: Modified Bis-Oxazoline Ligands" TETRAHEDRON LETTERS, ELSEVIER SCIENCE PUBLISHERS, AMSTERDAM, NL, vol. 32, no. 50, 1991, pages 7373-7376, XP002087674 ISSN: 0040-4039 * table 1 * | 5-11 | TECHNICAL FIELDS SEARCHED (Int.Cl.7) C07C |
| A | US 3 739 019 A (UEDA K ET AL) 12 June 1973 (1973-06-12) * example 1 * | 1-11 | |

The present search report has been drawn up for all claims

| Place of search | Date of completion of the search | Examiner |
|---|---|---|
| THE HAGUE | 5 June 2002 | O'Sullivan, P |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 02 00 4412

This annex lists the patent family members relating to the patent documents cited in the above-mentioned European search report.
The members are as contained in the European Patent Office EDP file on
The European Patent Office is in no way liable for these particulars which are merely given for the purpose of information.

05-06-2002

| Patent document cited in search report | Publication date | Patent family member(s) | Publication date |
|---|---|---|---|
| EP 0933349 A | 04-08-1999 | CN 1232017 A | 20-10-1999 |
| | | EP 0933349 A1 | 04-08-1999 |
| | | JP 11279111 A | 12-10-1999 |
| | | US 6268525 B1 | 31-07-2001 |
| US 3739019 A | 12-06-1973 | BE 752813 A1 | 16-12-1970 |
| | | CH 545262 A | 15-12-1973 |
| | | DE 2032097 A1 | 21-01-1971 |
| | | FR 2053984 A5 | 16-04-1971 |
| | | GB 1261469 A | 26-01-1972 |
| | | NL 7009751 A ,B | 05-01-1971 |

EPO FORM P0459

For more details about this annex : see Official Journal of the European Patent Office, No. 12/82